# EUROPÄISCHE PATENTSCHRIFT

(11) **EP 2 595 999 B1**
(45) Veröffentlichungstag und Bekanntmachung des Hinweises auf die Patenterteilung: **21.05.2014**
(21) Anmeldenummer: 11738673.0
(22) Anmeldetag: 12.07.2011
(51) Int. Cl.: C07F 5/00, C23C 18/12

(54) **INDIUMOXOALKOXIDE FÜR DIE HERSTELLUNG INDIUMOXID-HALTIGER SCHICHTEN**
INDIUM OXOALKOXIDES FOR PRODUCING COATINGS CONTAINING INDIUM OXIDE
OXO-ALCOXYDES D'INDIUM UTILISÉS POUR PRODUIRE DES COUCHES CONTENANT DE L'OXYDE D'INDIUM

(30) Priorität: 21.07.2010 DE 102010031895
(43) Veröffentlichungstag der Anmeldung: 29.05.2013
(73) Patentinhaber: Evonik Degussa GmbH, 45128 Essen (DE)
(72) Erfinder: STEIGER, Jürgen, 40237 Düsseldorf (DE); PHAM, Duy Vu, 46047 Oberhausen (DE); THIEM, Heiko, 64625 Bensheim (DE); MERKULOV, Alexey, 67059 Ludwigshafen (DE); HOPPE, Arne, 44623 Herne (DE)
(86) Internationale Anmeldenummer: PCT/EP2011/061867
(87) Internationale Veröffentlichungsnummer: WO 2012/010464

(56) Entgegenhaltungen:
- HYUN SUNG KIM ET AL: "High Performance Solution-Processed Indium Oxide Thin-Film Transistors", JOURNAL OF THE AMERICAN CHEMICAL SOCIETY, AMERICAN CHEMICAL SOCIETY, WASHINGTON, DC; US, Bd. 130, 24. September 2008 (2008-09-24), Seiten 12580-12581, XP002613079, ISSN: 0002-7863, DOI: 10.1021/JA804262Z [gefunden am 2008-08-29] in der Anmeldung erwähnt
- NAVABEH NAMI CHAMAZI ET AL: "Reaktionen von MeInCl2 mit Lithiumalkoholaten", ZEITSCHRIFT F&#XFFFD;R ANORGANISCHE UND ALLGEMEINE CHEMIE, Bd. 633, Nr. 13-14, 1. Oktober 2007 (2007-10-01), Seiten 2154-2158, XP55008632, ISSN: 0044-2313, DOI: 10.1002/zaac.200700017

## Beschreibung

Die vorliegende Erfindung betrifft Indiumoxoalkoxide für die Herstellung Indiumoxid-haltiger Schichten, Verfahren zu ihrer Herstellung und ihre Verwendung, insbesondere zur Herstellung Indiumoxid-haltiger Schichten, als Bestandteil von Beschichtungs-zusammensetzungen und zur Herstellung elektronischer Bauteile.

Indiumoxid (Indium(III)-oxid, In₂O₃) ist aufgrund der großen Bandlücke zwischen 3.6 und 3.75 eV (gemessen für aufgedampfte Schichten) [H.S. Kim, P.D. Byrne, A. Facchetti, T.J. Marks; J. Am. Chem. Soc. 2008, 130, 12580-12581] ein vielversprechender Halbleiter. Dünne Filme von wenigen hundert Nanometern Dicke können darüber hinaus eine hohe Transparenz im sichtbaren Spektralbereich von größer als 90 % bei 550 nm aufweisen. In extrem hoch geordneten Indiumoxid-Einkristallen kann man zudem Ladungsträgerbeweglichkeiten von bis zu 160 cm²/Vs messen.

Indiumoxid wird oft vor allem zusammen mit Zinn(IV)-oxid (SnO₂) als halbleitendes Mischoxid ITO eingesetzt. Aufgrund der verhältnismäßig hohen Leitfähigkeit von ITO-Schichten bei gleichzeitiger Transparenz im sichtbaren Spektralbereich findet es unter anderem Anwendung im Bereich von Flüssigkristallbildschirmen (LCD; liquid crystal display), insbesondere als "durchsichtige Elektrode". Diese zumeist dotierten Metalloxid-Schichten werden industriell vor allem durch kostenintensive Aufdampfmethoden im Hochvakuum hergestellt.

Indiumoxid-haltige Schichten und ihre Herstellung, insbesondere ITO-Schichten und reine Indiumoxid-Schichten, sowie ihre Herstellung sind somit von großer Bedeutung für die Halbleiter- und Displayindustrie.

Als mögliche Edukte bzw. Precursoren für die Synthese Indiumoxid-haltiger Schichten wird eine Vielzahl von Verbindungsklassen diskutiert. Zu diesen gehören zum Beispiel Indiumsalze. So beschreiben Marks et al. Bauteile, bei deren Herstellung eine Precursorlösung aus InCl₃ sowie der Base Monoethanolamin (MEA) gelöst in Methoxyethanol eingesetzt wird. Nach Aufschleudern (Spin-coating) der Lösung wird die entsprechende Indiumoxid-Schicht durch eine thermische Behandlung bei 400 °C erzeugt. [H.S. Kim, P.D. Byrne, A. Facchetti, T.J. Marks; J. Am. Chem. Soc. 2008, 130, 12580-12581 and supplemental informations]

An anderer Stelle werden als mögliche Edukte bzw. Precursoren für die Indiumoxid-Synthese Indiumalkoxide diskutiert. Unter einem Indiumalkoxid ist dabei eine Verbindung bestehend aus mindestens einem Indiumatom, mindestens einem Alkoxidrest der Formel - OR (R = organischer Rest) sowie ggf. einem oder mehreren organischen Resten -R, einem oder mehreren Halogenresten und/oder einem oder mehreren Resten -OH oder -OROH zu verstehen.

Im Stand der Technik sind unabhängig von einem möglichen Einsatz für die Indiumoxidbildung verschiedene Indiumalkoxide und Indiumoxoalkoxide beschrieben. Gegenüber den bereits erwähnten Indiumalkoxiden weisen Indiumoxoalkoxide noch mindestens einen weiteren, direkt an ein Indiumatom gebundenen oder mindestens zwei Indiumatome verbrückenden Sauerstoff-Rest (Oxo-Rest) auf.

Mehrotra et al. beschreiben die Herstellung von Indium-tris-Alkoxid In(OR)₃ aus Indium(III)-chlorid (InCl₃) mit Na-OR, wobei R für-Methyl, -Ethyl, iso-Propyl, n-, s-, t-Butyl und -Pentyl Reste steht. [S. Chatterjee, S. R. Bindal, R.C. Mehrotra; J. Indian Chem. Soc.1976, 53, 867].

Ein Review-Artikel von Carmalt et al. (Coordination Chemistry Reviews 250 (2006), 682-709) beschreibt verschiedene Gallium(III)- und Indium(III)-alkoxide und -aryloxide, die zum Teil auch über Alkoxidgruppen verbrückt vorliegen können. Weiterhin wird ein Oxozentrierter Cluster der Formel In₅(µ-O)(OⁱPr)₁₃, präziser [In₅(µ₅-O)(µ₃-OⁱPr)₄(µ₂-OⁱPr)₄(OⁱPr)₅] vorgestellt, bei dem es sich um ein Oxo-Alkoxid handelt und der nicht aus [In(OⁱPr)₃] hergestellt werden kann.

Ein Review-Artikel von N.Turova et al., Russian Chemical Reviews 73 (11), 1041-1064 (2004) fasst Synthese, Eigenschaften und Strukturen von Metalloxoalkoxiden, die dort als Precursoren für die Herstellung von oxidischen Materialien über Sol-Gel-Technologie betrachtet werden, zusammen. Neben einer Vielzahl anderer Verbindungen wird die Synthese und Struktur von [Sn₃O(OⁱBu)₁₀(ⁱBuOH)₂], von der bereits erwähnten Verbindung [In₅O(OⁱPr)₁₃] und von [Sn₆O₄(OR)₄] (R = Me, Prⁱ) beschrieben.

Der Artikel von N. Turova et al., Journal of Sol-Gel Science and Technology, 2, 17-23 (1994) präsentiert Ergebnisse von Studien an Alkoxiden, die dort als wissenschaftliche Basis für die Entwicklung von Sol-Gel-Prozessen von Alkoxiden und Alkoxid-basierten Pulvern betrachtet werden. In diesem Kontext wird auch u. a. auf ein vermeintliches "Indiumisopropoxid" eingegangen, das sich als das auch bei Carmalt et al. beschriebene Oxoalkoxid mit einem zentralen Sauerstoffatom und fünf umgebenden Metallatomen der Formel M₅(µ-O)(OⁱPr)₁₃ erwies.

Eine Synthese dieser Verbindung und ihre Kristallstruktur wird von Bradley et al., J. Chem. Soc., Chem. Commun., 1988, 1258 - 1259 beschrieben. Weitere Studien der Autoren führten zu dem Ergebnis, dass die Bildung dieser Verbindung nicht auf eine Hydrolyse von zwischenzeitlich entstandenem In(OⁱPr)₃ zurückzuführen ist (Bradley et al., Polyhedron Vol. 9, No. 5, pp. 719 - 726, 1990). Suh et al., J. Am. Chem. Soc. 2000, 122, 9396 - 9404 stellten weiterhin fest, dass diese Verbindung auch nicht auf thermischem Wege aus In(OⁱPr)₃ herstellbar ist. Außerdem wurde durch Bradley (Bradley et al., Polyhedron Vol. 9, No. 5, pp. 719 - 726, 1990) festgestellt, dass sich diese Verbindung nicht sublimieren lässt.

Metalloxidschichten lassen sich prinzipiell über verschiedene Verfahren herstellen.

Eine Möglichkeit, Metalloxidschichten herzustellen, basiert auf Sputtertechniken. Diese Techniken haben jedoch den Nachteil, dass sie unter Hochvakuum durchgeführt werden müssen. Ein weiterer Nachteil ist, dass die mit ihnen hergestellten Filme viele Sauerstoff-Defekte aufweisen, die das Einstellen einer gezielten und reproduzierbaren Stöchiometrie der Schichten unmöglich machen und somit zu schlechten Eigenschaften der hergestellten Schichten führen.

Eine andere prinzipielle Möglichkeit zur Herstellung von Metalloxidschichten beruht auf chemischer Gasphasenabscheidung. So können zum Beispiel Indiumoxid-haltige Schichten aus Indiumoxid-Precursoren wie Indiumalkoxiden oder Indiumoxoalkoxiden über Gasphasenabscheidung hergestellt werden. So lehrt z.B. US 6,958,300 B2, mindestens einen Metall-Organo-Oxid-Precursor (Alkoxid bzw. Oxoalkoxid) der generischen Formel M¹_{q}(O)ₓ(OR¹)_{y} (q = 1 - 2; x = 0 - 4, y = 1 - 8, M¹ = Metall; z.B. Ga, In oder Zn, R¹ = organischer Rest; Alkoxid für x = 0, Oxo-Alkoxid für ≥ 1) bei der Herstellung von Halbleitern bzw. Metalloxidschichten durch Gasphasenabscheidung wie z.B. CVD oder ALD einzusetzen. Alle Gasphasenabscheidungsprozesse haben jedoch entweder den Nachteil, dass sie i) im Falle einer thermischen Reaktionsführung den Einsatz sehr hoher Temperaturen oder ii) im Falle des Einbringens der erforderlichen Energie für die Zersetzung des Precursors in Form von elektromagnetischer Strahlung hohe Energiedichten erfordern. In beiden Fällen ist es nur mit höchstem apparativem Aufwand möglich, die zur Zersetzung des Precursors benötigte Energie gezielt und einheitlich einzubringen.

Vorteilhaft werden somit Metalloxidschichten über Flüssigphasen-Verfahren hergestellt, d.h. über Verfahren umfassend mindestens einen Verfahrensschritt vor der Konvertierung zum Metalloxid, bei dem das zu beschichtende Substrat mit einer flüssigen Lösung von mindestens einem Precursor des Metalloxids beschichtet, ggf. nachfolgend getrocknet, und konvertiert wird. Unter einem Metalloxid-Precursor ist dabei eine thermisch oder mit elektromagnetischer Strahlung zersetzbare Verbindung, mit der in An- oder Abwesenheit von Sauerstoff oder anderen Oxidationsstoffen Metalloxid-haltige Schichten gebildet werden können, zu verstehen. Prominente Beispiele für Metalloxid-Precursoren sind z.B. Metallalkoxide. Prinzipiell kann die Schichtherstellung dabei i) durch Sol-Gel-Prozesse, bei denen die eingesetzten Metallalkoxide in Gegenwart von Wasser durch Hydrolyse und nachfolgende Kondensation zunächst zu Gelen umgesetzt werden und dann in Metalloxide konvertiert werden, oder ii) aus nichtwässriger Lösung erfolgen.

Dabei gehört auch die Herstellung Indiumoxid-haltiger Schichten aus Indiumalkoxiden aus flüssiger Phase zum Stand der Technik.

Die Herstellung Indiumoxid-haltiger Schichten aus Indiumalkoxiden über Sol-Gel-Verfahren in Gegenwart signifikanter Mengen von Wasser gehört zum Stand der Technik. WO 2008/083310 A1 beschreibt Verfahren zur Herstellung anorganischer Schichten bzw. organischer/anorganischer Hybridschichten auf einem Substrat, bei dem ein Metallalkoxid (z.B. eines der generischen Formel R¹M-(OR²)_{y-x}) oder ein Präpolymer davon auf ein Substrat aufgebracht und dann die resultierende Metallalkoxid-Schicht in Gegenwart von und Reaktion mit Wasser ausgehärtet wird. Bei den einsetzbaren Metallalkoxiden kann es sich u.a. um solche von Indium, Gallium, Zinn oder Zink handeln. Nachteilig an dem Einsatz von Sol-Gel-Verfahren ist jedoch, dass die Hydrolyse-Kondensations-Reaktion automatisch durch Wasserzugabe gestartet wird und nach ihrem Start nur schlecht kontrollierbar ist. Wird der Hydrolyse-Kondensations-Prozess bereits vor dem Aufbringen auf das Substrat gestartet, sind die zwischenzeitlich erzeugten Gele aufgrund ihrer erhöhten Viskosität oft für Verfahren zum Erzeugen feiner Oxidschichten nicht geeignet. Wird der Hydrolyse-Kondensations-Prozess dagegen erst nach dem Aufbringen auf das Substrat durch Zufuhr von Wasser in flüssiger Form oder als Dampf gestartet, führen die so resultierenden schlecht durchmischten und inhomogenen Gele oft zu entsprechend inhomogenen Schichten mit nachteiligen Eigenschaften.

JP 2007-042689 A beschreibt Metallalkoxid-Lösungen, die Indiumalkoxide enthalten können, sowie Verfahren zur Herstellung von Halbleiterbauelementen, die diese Metallalkoxid-Lösungen einsetzen. Die Metallalkoxid-Filme werden thermisch behandelt und zur OxidSchicht umgewandelt, auch diese Systeme liefern jedoch nicht ausreichend homogene Filme. Reine Indiumoxid-Schichten können jedoch mit dem dort beschriebenen Verfahren nicht hergestellt werden.

Die noch nicht offen gelegte DE 10 2009 009 338 beschreibt den Einsatz von Indiumalkoxiden bei der Herstellung von Indiumoxid-haltigen Schichten aus wasserfreien Lösungen. Die resultierenden Schichten sind zwar homogener als bei über Sol-Gel-Prozesse hergestellten Schichten, der Einsatz von Indiumalkoxiden in wasserfreien Systemen hat jedoch immer noch den Nachteil, dass durch die Konvertierung Indiumalkoxid-haltiger Formulierungen zu Indiumoxid-haltigen Schichten keine ausreichend gute elektrische Performance der entstehenden Schicht gegeben ist.

Die ebenfalls noch nicht offen gelegte DE 10 2009 028 801 beschreibt Flüssigphasen-Verfahren zur Herstellung demgegenüber verbesserter Indiumoxid-haltiger Schichten aus nichtwässriger Lösung, bei dem eine wasserfreie Zusammensetzung enthaltend i) mindestens ein Indiumoxoalkoxid der generischen Formel MₓO_{y}(OR)_{z}[O(R'O)_{c}H]ₐX_{b}[R"OH]_{d} mit M = In, x = 3 - 25, y = 1 - 10, z = 3 - 50 , a = 0 - 25, b = 0 - 20, c = 0 - 1, d = 0 - 25, R, R', R" = organischer Rest, X = F, Cl, Br, I und ii) mindestens ein Lösemittel auf ein Substrat aufgebracht, ggf. getrocknet, und in eine Indiumoxid-haltige Schicht konvertiert wird.

Nichtsdestotrotz ist die Erzeugung noch besserer Indiumoxid-haltiger Schichten wünschenswert. Es ist somit die Aufgabe der vorliegenden Erfindung, Substanzen bereitzustellen, die zur Erzeugung von Indiumoxid-haltigen Schichten (insbesondere von Indiumoxidschichten) mit noch besserer elektrischer Performance (insbesondere noch besseren Feldeffektbeweglichkeiten µ_{FET}) eingesetzt werden können.

Diese Aufgabe wird vorliegend durch das erfindungsgemäße halogenhaltige Indiumoxoalkoxid der generischen Formel In₇O₂(OH)(OR)₁₂X₄(ROH)ₓ mit R = C1-C15-Alkyl, C1-C15-Alkenyl, C1-C15-Alkinyl, C1-C15-Alkoxyalkyl, C6-C15-Aryl- oder C7-C15-Alkoxyaryl, X = F, Cl, Br, I und x = 0 bis 10 gelöst. Überraschenderweise können weiterhin mit diesen Substanzen besonders gut qualitativ hochwertige Indiumoxidhaltige Schichten an Luft hergestellt werden.

Diese bislang in der Literatur nicht beschriebenen Verbindungen In₇O₂(OH)(OR)₁₂X₄(ROH)ₓ können über eine Umsetzung eines Reaktionsgemisches (A) umfassend ein Indium(III)-halogenid InX₃, ein Natrium- oder Kaliumisopropanolat und (bevorzugt getrocknetes) Isopropanol, bevorzugtes Abfiltrieren zwischenzeitlich entstandenen Feststoffes (F1) und Waschung mit Isopropanol unter Erhalt Isopropanol-haltiger Waschlösungen (B), Entfernen des Isopropanols aus den Isopropanol-haltigen Lösungen (A) und/oder (B) unter Erhalt eines Feststoffes (F2), Aufnahme des Feststoffes (F2) in dem Alkohol ROH unter Erhalt eines Gemisches (C), bevorzugte Durchführung einer oder mehrerer Filtrationsschritte, und Kristallisation der Indiumoxoalkoxide aus dem Gemisch (C) synthetisiert werden.

Im erfindungsgemäßen Verfahren beträgt das molare Verhältnis von eingesetztem Natrium- oder Kaliumisopropanolat zu Indium(III)-halogenid InX₃ vorzugsweise 2,0 bis 2,51 : 1, insbesondere 2,4 bis 2,5 : 1.

Weiterhin bevorzugt ist das molare Verhältnis von dem eingesetzten Isopropanol und dem eingesetzten Indium(III)-halogenid InX₃ 5 bis 3000 : 1, , ganz besonderes bevorzugt 15 bis 100 : 1.

Bevorzugte Indiumoxoalkoxide sind die entsprechenden Chloride, d.h. entsprechende Verbindungen, in denen X = Cl ist. Diese Verbindungen führen zu besonders guten elektrischen Eigenschaften der entsprechenden Indiumoxid-haltigen Schicht und lassen sich besonders einfach herstellen.

Ebenfalls bevorzugt sind entsprechende Indiumoxoalkoxide, in denen R = -CH₃, -CH₂CH₃,-CH₂CH₂CH₃, -CH(CH₃)₂, -CH₂CH₂CH₂CH₃, -CH(CH3₎(CH₂CH₃), -CH₂CH₂(CH₃)₂ oder -C(CH₃)₃ ist und die besonders einfach herstellbar und prozessierbar sind.

Außerdem bevorzugt sind entsprechende Indiumoxoalkoxyalkoxide, in denen R = -CH₂CH₂-OCH₃, -CH₂CH₂-OCH₂CH₃, -CH₂CH₂-OCH₂CH₂CH₃, -CH₂CH₂-OCH(CH₃)₂, -CH₂CH₂-OCH₂CH₂CH₂CH₃, -CH₂CH₂-OCH₂CH(CH₃)₂, -CH₂CH₂-OCH(CH₃)(CH₂CH₃) oder -CH₂CH₂-OC(CH₃)₃ ist und die besonders einfach herstellbar und prozessierbar sind.

Besonders gute Ausbeuten bei dem erfindungsgemäßen Verfahren resultieren, wenn das beschriebene Verfahren mit InCl₃ und Alkoholen ausgewählt aus der Gruppe bestehend aus CH₃OH, HOCH₂CH₃, HOCH₂CH₂CH₃, HOCH(CH₃)₂, HOCH₂CH₂CH₂CH₃, HOCH(CH₃)(CH₂CH₃), HOCH₂CH₂(CH₃)₂, oder HOC(CH₃)₃ durchgeführt wird. Die entstehenden bevorzugten Verfahrensprodukte sind In₇O₂(OH)(OCH₃)₁₂Cl₄(CH₃OH)ₓ, In₇O₂(OH)(OCH₂CH₃)₁₂Cl₄(CH₃CH₂OH)ₓ, In₇O₂(OH)(OCH₂CH₂CH₃)₁₂Cl₄(CH₃CH₂CH₂OH)ₓ, In₇O₂(OH)(OCH(CH₃)₂)₁₂Cl₄(HOCH(CH₃)₂)ₓ, In₇O₂(OH)(OCH₂CH₂CH₂CH₃)₁₂Cl₄(HOCH₂CH₂CH₂CH₃)ₓ, In₇O₂(OH)(OCH(CH₃)(CH₂CH₃))₁₂Cl₄(HOCH(CH₃)(CH₂CH₃))ₓ, und In₇O₂(OH)(OC(CH₃)₃)₁₂Cl₄(HOC(CH₃)₃)ₓ mit jeweils x = 0 - 10.

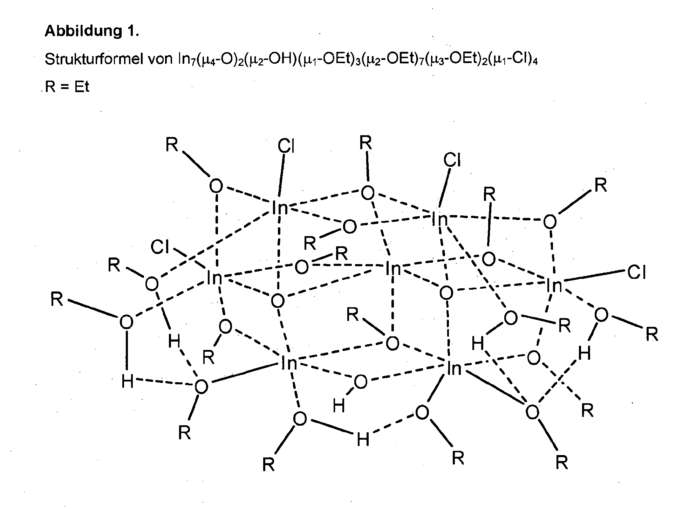

Ein besonders bevorzugtes erfindungsgemäßes Indiumoxoalkoxid ist ein Indiumoxoalkoxid der generischen Formel In₇(µ₄-O)₂(µ₂-OH)(µ₁-OEt)₃(µ₂-OEt)₇(µ₃-OEt)₂(µ₁-Cl)₄, das die in Abbildung 1 dargestellte Strukturformel aufweist und das weiterhin mit bis zu 10 Ethanolmolekülen koordiniert im Kristall vorliegen kann.

Diese Verbindung kann wie im beigefügten Beispiel beschrieben hergestellt werden.

Gegenstand der vorliegenden Erfindung ist weiterhin die Verwendung der erfindungsgemäßen Indiumalkoxide zur Herstellung Indiumoxid-haltiger Schichten sowie entsprechende Verfahren zur Herstellung Indiumoxid-haltiger Schichten. Dabei eignet sich das erfindungsgemäße Indiumoxoalkoxid prinzipiell für die Schichtherstellung über Sputterprozesse, über Gasphasenabscheidung, für Sol-Gel-Verfahren und für die Abscheidung aus nichtwässriger Lösung. Besonders gut geeignet ist das erfindungsgemäße Indiumoxoalkoxid, weiter bevorzugt die Indiumoxoalkoxide der generischen Formeln sind In₇O₂(OH)(OCH₃)₁₂Cl₄(CH₃OH)ₓ, In₇O₂(OH)(OCH₂CH₃)₁₂Cl₄(CH₃CH₂OH)ₓ, In₇O₂(OH)(OCH₂CH₂CH₃)₁₂Cl₄(CH₃CH₂CH₂OH)ₓ, In₇O₂(OH)(OCH(CH₃)₂)₁₂Cl₄(HOCH(CH₃)₂)ₓ, In₇O₂(OH)(OCH₂CH₂CH₂CH₃)₁₂Cl₄(HOCH₂CH₂CH₂CH₃)ₓ, In₇O₂(OH)(OCH(CH₃)(CH₂CH₃))₁₂Cl₄(HOCH(CH₃)(CH₂CH₃))ₓ, und In₇O₂(OH)(OC(CH₃)₃)₁₂Cl₄(HOC(CH₃)₃)ₓ mit x = 0 - 10 und ganz besonders das bevorzugte Indiumoxoalkoxid der Formel In₇(µ₄-O)₂(µ₂-OH)(µ₁-OEt)₃(µ₂-OEt)₇(µ₃-OEt)₂(µ₁-Cl)₄(µ₁-EtOH)ₓ mit x = 0 - 10 jedoch für die Abscheidung aus nichtwässriger Lösung.

Bei einem solchen Verfahren zur Herstellung Indiumoxid-haltiger Schichten über Abscheidung aus nichtwässriger Lösung handelt es sich um ein Verfahren, bei dem das zu beschichtende Substrat mit einer flüssigen nichtwässrigen Lösung enthaltend mindestens ein erfindungsgemäßes halogenhaltiges Indiumoxoalkoxid beschichtet, ggf. nachfolgend getrocknet, und dann in eine Indiumoxid-haltige Schicht umgewandelt wird. Unter flüssigen Zusammensetzungen im Sinne der vorliegenden Erfindung sind solche zu verstehen, die bei SATP-Bedingungen ("Standard Ambient Temperature and Pressure"; T = 25 °C und p = 1013 hPa) und bei Aufbringen auf das zu beschichtende Substrat flüssig vorliegen. Unter einer nichtwässrigen Lösung bzw. einer wasserfreien Zusammensetzung ist dabei hier und im Folgenden eine Lösung bzw. Formulierung zu verstehen, die nicht mehr als 200 ppm H₂O aufweist.

Unter dem Verfahrensprodukt des erfindungsgemäßen Verfahrens, der Indiumoxid-haltigen Schicht, ist eine metall- bzw. halbmetallhaltige Schicht zu verstehen, die Indiumatome bzw.-ionen aufweist, die im Wesentlichen oxidisch vorliegen. Gegebenenfalls kann die Indiumoxid-haltige Schicht auch noch Kohlenstoff-, Stickstoff-, Halogen- oder Alkoxid-Anteile aus einer nicht vollständigen Konvertierung oder einer unvollständigen Entfernung entstehender Nebenprodukte aufweisen. Die Indiumoxid-haltige Schicht kann dabei eine reine Indiumoxid-Schicht sein, d.h. bei Nichtberücksichtigung etwaiger Kohlenstoff-, Stickstoff-, Alkoxid- oder Halogen-Anteile im Wesentlichen aus oxidisch vorliegenden Indiumatome bzw. -ionen bestehen, oder anteilig noch weitere Elemente, die selbst in elementarer, oxidischer oder anderer Form vorliegen können, aufweise. Zur Erzeugung reiner Indiumoxid-Schichten sollten bei dem erfindungsgemäßen Verfahren nur Indium-haltige Precursoren, bevorzugt neben dem erfindungsgemäßen mindestens einen Indiumoxoalkoxid nur Indiumoxoalkoxide und Indiumalkoxide, eingesetzt werden. Im Gegensatz dazu sind zur Erzeugung auch andere Metalle aufweisender Schichten neben den Indium-haltigen Precursoren auch Precursoren von Metallen in der Oxidationsstufe 0 (zur Herstellung von Schichten enthaltend weitere Metalle in neutraler Form) bzw. Metalloxid-Precursoren (wie z.B. andere Metallalkoxide oder-oxoalkoxide) einzusetzen.

Das vorliegende erfindungsgemäße Verfahren ist besonders gut geeignet zur Herstellung von Indiumoxidschichten, wenn das erfindungsgemäße Indiumoxoalkoxid als einziger Metalloxid-Precursor, eingesetzt wird. Ganz besonders gute Schichten resultieren, wenn der einzige Metalloxid-Precursor der Formel In₇(µ₄-O)₂(µ₂-OH)(µ₁-OEt)₃(µ₂-OEt)₇(µ₃-OEt)₂(µ₁-Cl)₄ entspricht.

Das mindestens eine Indiumoxoalkoxid liegt bevorzugt in Anteilen von 0,1 bis 15 Gew.-%, besonders bevorzugt 1 bis 10 Gew.-%, ganz besonders bevorzugt 2 bis 5 Gew.-% bezogen auf die Gesamtmasse der wasserfreien Zusammensetzung vor.

Die wasserfreie Zusammensetzung enthält weiterhin mindestens ein Lösemittel, d.h. die Zusammensetzung kann sowohl ein Lösemittel oder ein Gemisch verschiedener Lösemittel enthalten. Vorzugsweise für das erfindungsgemäße Verfahren in der Formulierung einsetzbar sind aprotische und schwach protische Lösemittel, d.h. solche ausgewählt aus der Gruppe der aprotischen unpolaren Lösemittel, d.h. der Alkane, substituierten Alkane, Alkene, Alkine, Aromaten ohne oder mit aliphatischen oder aromatischen Substituenten, halogenierten Kohlenwasserstoffe, Tetramethylsilan, der Gruppe der aprotischen polaren Lösemittel, d.h. der Ether, aromatischen Ether, substituierten Ether, Ester oder Säureanhydride, Ketone, tertiäre Amine, Nitromethan, DMF (Dimethylformamid), DMSO (Dimethylsulfoxid) oder Propylencarbonat und der schwach protischen Lösemittel, d.h. der Alkohole, der primären und sekundären Amine und Formamid. Besonders bevorzugt einsetzbare Lösemittel sind Alkohole sowie Toluol, Xylol, Anisol, Mesitylen, n-Hexan, n-Heptan, Tris-(3,6-dioxaheptyl)-amin (TDA), 2-Aminomethyltetrahydrofuran, Phenetol, 4-Methylanisol, 3-Methylanisol, Methylbenzoat, Ethylbenzoat, Ethyllactat, Butylacetat, N-Methyl-2-pyrrolidon (NMP), Tetralin, und Diethylether. Ganz besonders bevorzugte Lösemittel sind Methanol, Ethanol, Isopropanol, Tetrahydrofurfurylalkohol, tert-Butanol, n-Butanol, Ethyllactat, Butylacetat, und Toluol sowie ihre Gemische.

Bevorzugt weist die bei dem erfindungsgemäßen Verfahren eingesetzte Zusammensetzung zur Erzielung einer besonders guten Verdruckbarkeit eine Viskosität von 1 mPa·s bis 10 Pa.s, insbesondere 1 mPa·s bis 100 mPa·s bestimmt nach DIN 53019 Teil 1 bis 2 und gemessen bei 20 °C auf. Entsprechende Viskositäten können durch Zugabe von Polymeren, Cellulosederivaten, oder z.B. unter der Handelsbezeichnung Aerosil erhältlichem SiO₂, und insbesondere durch PMMA, Polyvinylalkohol, Urethanverdicker oder Polyacrylatverdicker eingestellt werden.

Bei dem Substrat, das bei dem erfindungsgemäßen Verfahren eingesetzt wird, handelt es sich bevorzugt um ein Substrat bestehend aus Glas, Silicium, Siliciumdioxid, einem Metalloder Übergangsmetalloxid, einem Metall oder einem polymeren Material, insbesondere PI oder PET.

Die Herstellung Indiumoxid-haltiger Schichten erfolgt bevorzugt über ein Beschichtungsverfahren ausgewählt aus Druckverfahren (insbesondere Flexo/Gravur-Druck, Inkjet-Druck, Offset-Druck, digitalem Offset-Druck und Siebdruck), Sprühverfahren, Rotationsbeschichtungsverfahren ("Spin-coating"), Tauchverfahren ("Dip-coating") und Verfahren ausgewählt aus Meniscus Coating, Slit Coating, Slot-Die Coating, und Curtain Coating. Ganz besonders bevorzugt ist das Beschichtungsverfahren ein Druckverfahren.

Nach der Beschichtung und vor der Konvertierung kann das beschichtete Substrat weiterhin getrocknet werden. Entsprechende Maßnahmen und Bedingungen hierfür sind dem Fachmann bekannt.

Die Umwandlung zu einer Indiumoxid-haltigen Schicht kann auf thermischem Wege und/oder durch Bestrahlung mit elektromagnetischer, insbesondere aktinischer Strahlung erfolgen. Bevorzugt erfolgt die Konvertierung auf dem thermischen Wege durch Temperaturen von größer als 150 °C. Besonders gute Ergebnisse können jedoch erzielt werden, wenn zur Konvertierung Temperaturen von 250 °C bis 360 °C eingesetzt werden.

Dabei werden typischerweise Konvertierungszeiten von einigen Sekunden bis hin zu mehreren Stunden verwendet.

Die thermische Konvertierung kann weiterhin dadurch unterstützt werden, dass vor, während oder nach der thermischen Behandlung UV-, IR- oder VIS-Strahlung eingestrahlt oder das beschichtete Substrat mit Luft bzw. Sauerstoff behandelt wird.

Die Güte der nach dem erfindungsgemäßen Verfahren erzeugten Schicht kann weiterhin durch eine an den Konvertierungsschritt anschließende kombinierte Temperatur- und Gasbehandlung (mit H₂ oder O₂), Plasmabehandlung (Ar-, N₂-, O₂- oder H₂-Plasma), Laser-Behandlung (mit Wellenlängen im UV-, VIS- oder IR-Bereich) oder eine Ozon-Behandlung weiter verbessert werden.

Gegenstand der Erfindung sind weiterhin über die aus den erfindungsgemäßen Indiumoxoalkoxiden herstellbaren Indiumoxid-haltigen Schichten. Besonders gute Eigenschaften haben dabei aus den erfindungsgemäßen Indiumoxoalkoxiden herstellbare Indiumoxid-haltige Schichten, die reine Indiumoxidschichten sind.

Aufgrund der guten Eignung der erfindungsgemäßen Indiumoxoalkoxide für die Herstellung Indiumoxid-haltiger Schichten aus flüssiger Phase ist ein weiterer Gegenstand der vorliegenden Erfindung die Verwendung der erfindungsgemäßen Indiumoxoalkoxide zur Herstellung flüssiger Beschichtungszusammensetzungen. Unter flüssigen Beschichtungszusammensetzungen sind dabei solche zu verstehen, die bei SATP-Bedingungen ("Standard Ambient Temperature and Pressure"; T = 25 °C und p = 1013 hPa) und bei Aufbringen auf das zu beschichtende Substrat flüssig vorliegen.

Die erfindungsgemäßen Indiumoxoalkoxide eignen sich weiterhin vorteilhaft für die Herstellung elektronischer Bauteile, insbesondere die Herstellung von Transistoren (insbesondere Dünnschichttransistoren), Displays, Funketiketten, Schaltungen, Dioden, Sensoren oder Solarzellen.

Die nachfolgenden Beispiele sollen den Gegenstand der vorliegenden Erfindung ohne Einschränkung näher erläutern.

### Ausführungsbeispiele:

### Synthese von In₇(µ₄-O)₂(µ₂-OH)(µ₁-OEt)₃(µ₂-OEt)₇(µ₃-OEt)₂(µ₁-Cl)₄(µ₁-HOEt)₅

In einem von Restfeuchte befreiten 1L-Glasrundkolben wurden 1,3 g Natrium (56,55 mmol, 2,5 Äquivalenten) in 250 mL Isopropanol unter Schutzgasatmosphäre und Rückfluss aufgelöst. Zu der entstandenen NaOⁱPr-Lösung wurden 5,0 g (22.6 mmol) Indium(III)-chlorid (InCl₃) in 250 ml getrocknetem Isopropanol zugegeben. Das Reaktionsgemisch wurde zwei Stunden unter Rückfluss intensiv gerührt. Nach dem Abkühlen wurde die Lösung filtriert und der Niederschlag wurde zweimal mit jeweils 100 mL Isopropanol gewaschen. Die kombinierten flüssigen Fraktionen wurden im Vakuum eingeengt. Der zurückbleibende Feststoff wurde in 200 mL Ethanol (<10 ppm H₂O) aufgenommen und filtriert. Nach ca. 1 Woche wurden Kristalle aus der Lösung bei -30°C gebildet (Ausbeute ca. 20%). Laut Einkristallstrukturanalyse entspricht der Molekülaufbau im Kristall der Formel In₇(µ₄-O)₂(µ₂-OH)(µ₁-OEt)₃(µ₂-OEt)₇(µ₃-OEt)₂(µ₁-Cl)₄(µ₁-HOEt)₅.

### Herstellung von Indiumoxidschichten

Ein dotiertes Siliciumsubstrat mit einer Kantenlänge von etwa 15 mm und mit einer ca. 200 nm dicken Siliciumoxid-Beschichtung und Fingerstrukturen aus ITO/Gold wurde mit 100 µl einer 5 Gew.-% Lösung von In₇(µ₄-O)₂(µ₂-OH)(µ₁-OEt)₃(µ₂-OEt)₇(µ₃-OEt)₂(µ₁-Cl)₄(µ₁-HOEt)₅ in Alkohol (Methanol, Ethanol oder Isopropanol) oder Toluol per Spin-Coating (2000 rpm) beschichtet. Nach dem Beschichtungsvorgang wurde das beschichtete Substrat an der Luft bei einer Temperatur von 260°C oder 350 °C eine Stunde lang getempert.

Die erfindungsgemäße Beschichtung zeigen eine Ladungsträgerbeweglichkeit von bis zu 1.5 cm²/Vs (bei 30 V Gate-Source-Spannung, 30 V Source-Drain-Spannung, 1 cm Kanalbreite und 20 µm Kanallänge).

**Tabelle 1. Ladungsträgerbeweglichkeiten**

| | Ladungsträgerbeweglichkeit / cm²V⁻¹s⁻¹ | |
|---|---|---|
| Lösungsmittel | 260°C | 350°C |
| Methanol | 0.2 | 1.0 |
| Ethanol | 0.25 | 1.1 |
| Isopropanol | 0.5 | 1.5 |
| Toluol | 0.1 | 0.8 |

## Patentansprüche

1. Halogenhaltiges Indiumoxoalkoxid der generischen Formel
In₇O₂(OH)(OR)₁₂X₄(ROH)ₓ
mit
R = C1-C15-Alkyl, C1-C15-Alkenyl, C1-C15-Alkinyl, C1-C15-Alkoxyalkyl, C6-C15-Aryl- oder C7-C15-Alkoxyaryl,
X = F, Cl, Br, I und
x = 0 bis 10.

2. Indiumoxoalkoxid nach Anspruch 1,
**dadurch gekennzeichnet, dass** es sich durch die generische Formel
In₇(µ₄-O)₂(µ₂-OH)(µ₁-OEt)₃(µ₂-OEt)₇(µ₃-OEt)₂(µ₁-Cl)₄
beschreiben lässt.

3. Verfahren zur Herstellung von Indiumoxoalkoxiden gemäß Anspruch 1 oder 2,
**dadurch gekennzeichnet, dass**
- ein Reaktionsgemisch (A) umfassend ein Indium(III)-halogenid InX₃, ein Natrium- oder Kaliumisopropanolat und Isopropanol umgesetzt wird,
- bevorzugt zwischenzeitlich entstandener Feststoff (F1) abfiltriert und mit Isopropanol unter Erhalt einer Isopropanol-haltigen Waschlösung (B) gewaschen wird,
- aus den Isopropanol-haltigen Lösungen (A) und/oder (B) das Isopropanol unter Erhalt eines Feststoffes (F2) entfernt wird,
- der Feststoff (F2) in dem Alkohol ROH unter Erhalt eines Gemisches (C) aufgenommen wird,
- bevorzugt ein oder mehrere Filtrationsschritte durchgeführt werden,
- und die Indiumoxoalkoxide aus dem Gemisch (C) kristallisiert werden.

4. Verfahren zur Herstellung von Indiumoxoalkoxiden gemäß Anspruch 3, **dadurch gekennzeichnet, dass** das Indium(III)-halogenid InX₃ InCl₃ ist und der Alkohol ausgewählt aus der Gruppe bestehend aus CH₃OH, HOCH₂CH₃, HOCH₂CH₂CH₃, HOCH(CH₃)₂, HOCH₂CH₂CH₂CH₃, HOCH(CH₃)(CH₂CH₃) oder HOC(CH₃)₃ wird.

5. Verwendung eines Indiumoxoalkoxids gemäß Anspruch 1 oder 2 zur Herstellung Indiumoxid-haltiger Beschichtungen.

6. Verwendung eines Indiumoxoalkoxids gemäß Anspruch 5,
**dadurch gekennzeichnet, dass**
die Herstellung Indiumoxid-haltiger Beschichtungen über Abscheidung aus nichtwässriger Lösung erfolgt.

7. Verwendung eines Indiumoxoalkoxids gemäß einem der Ansprüche 1 oder 2 zur Herstellung flüssiger Beschichtungszusammensetzungen.

8. Verwendung eines Indiumoxoalkoxids gemäß einem der Ansprüche 1 oder 2 zur Herstellung elektronischer Bauteile, insbesondere zur Herstellung von Transistoren, Displays, Funketiketten, Schaltungen, Dioden, Sensoren oder Solarzellen.

## Claims

1. Halogenated indium oxo alkoxide of the generic formula
In₇O₂(OH)(OR)₁₂X₄(ROH)ₓ
where
R = C1-C15-alkyl, C1-C15-alkenyl, C1-C15-alkynyl, C1-C15-alkoxyalkyl, C6-C15-aryl- or C7-C15-alkoxyaryl,
X = F, Cl, Br, I and
x = 0 to 10.

2. Indium oxo alkoxide according to Claim 1, **characterized in that** it can be described by the generic formula
In₇(µ₄-O)₂(µ₂-OH)(µ₁-OEt)₃(µ₂-OEt)₇(µ₃-OEt)₂(µ₁-Cl)₄.

3. Process for preparing indium oxo alkoxides according to Claim 1 or 2, **characterized in that**
- a reaction mixture (A) comprising an indium(III) halide InX₃, a sodium or potassium isopropoxide and isopropanol is reacted,
- solid (F1) formed as an intermediate is preferably filtered off and washed with isopropanol to obtain an isopropanol-containing wash solution (B),
- the isopropanol is removed from the isopropanol-containing solutions (A) and/or (B) to obtain a solid (F2),
- the solid (F2) is taken up in the alcohol ROH to obtain a mixture (C),
- preferably one or more filtration steps are carried out,
- and the indium oxo alkoxides are crystallized out of the mixture (C).

4. Process for preparing indium oxo alkoxides according to Claim 3, **characterized in that** the indium(III) halide InX₃ is InCl₃ and the alcohol is selected from the group consisting of CH₃OH, HOCH₂CH₃, HOCH₂CH₂CH₃, HOCH(CH₃)₂, HOCH₂CH₂CH₂CH₃, HOCH(CH₃)(CH₂CH₃) and HOC(CH₃)₃.

5. Use of an indium oxo alkoxide according to Claim 1 or 2 for production of indium oxide-containing coatings.

6. Use of an indium oxo alkoxide according to Claim 5, **characterized in that**
the indium oxide-containing coatings are produced by means of deposition from nonaqueous solution.

7. Use of an indium oxo alkoxide according to either of Claims 1 and 2 for production of liquid coating compositions.

8. Use of an indium oxo alkoxide according to either of Claims 1 and 2 for production of electronic components, especially for production of transistors, displays, RFID tags, circuits, diodes, sensors or solar cells.

## Revendications

1. Oxoalcoolate d'indium halogéné de formule générale
In₇O₂(OH)(OR)₁₂X₄(ROH)ₓ
où
R = alkyle en C₁-C₁₅, alcényle en C₁-C₁₅, alcynyle en C₁-C₁₅, alcoxyalkyle en C₁-C₁₅, aryle en C₆-C₁₅ ou alcoxyaryle en C₇-C₁₅,
X = F, Cl, Br, I et
x 0 à 10.

2. Oxoalcoolate d'indium selon la revendication 1, **caractérisé en ce qu'**il peut être décrit par la formule générale In₇(µ₄-O)₂(µ₂-OH)(µ₁-OEt)₃(µ₂-OEt)₇(µ₃-OEt)₂(µ₁-Cl)₄.

3. Procédé pour la préparation d'oxoalcoolates d'indium selon la revendication 1 ou 2, **caractérisé en ce que**
- on fait réagir un mélange réactionnel (A) comprenant un halogénure d'indium(III) InX₃, un isopropanolate de sodium ou potassium et de l'isopropanol,
- de préférence on sépare par filtration le solide (F1) formé entre-temps et on le lave avec de l'isopropanol, avec obtention d'une solution de lavage (B) contenant de l'isopropanol,
- on élimine l'isopropanol des solutions (A) et/ou (B) contenant de l'isopropanol, avec obtention d'un solide (F2),
- on reprend le solide (F2) dans l'alcool ROH avec obtention d'un mélange (C),
- de préférence on effectue une ou plusieurs étapes de filtration,
- et on fait cristalliser les oxoalcoolates d'indium dans le mélange (C).

4. Procédé pour la préparation d'oxoalcoolates d'indium selon la revendication 3, **caractérisé en ce que** l'halogénure d'indium(III) InX₃ est InCl₃ et on choisit l'alcool dans le groupe constitué par CH₃OH, HOCH₂CH₃, HOCH₂CH₂CH₃, HOCH(CH₃)₂, HOCH₂CH₂CH₂CH₃, HOCH(CH₃)(CH₂CH₃) ou HOC (CH₃)₃.

5. Utilisation d'un oxoalcoolate d'indium selon la revendication 1 ou 2, pour la production de revêtements contenant de l'oxyde d'indium.

6. Utilisation d'un oxoalcoolate d'indium selon la revendication 5,
**caractérisée en ce que**
la production de revêtements contenant de l'oxyde d'indium s'effectue par dépôt à partir d'une solution non aqueuse.

7. Utilisation d'un oxoalcoolate d'indium selon l'une quelconque des revendications 1 et 2, pour la préparation de compositions liquides de revêtement.

8. Utilisation d'un oxoalcoolate d'indium selon l'une quelconque des revendications 1 et 2, pour la fabrication de composants électroniques, en particulier pour la fabrication de transistors, afficheurs, radio-étiquettes, circuits, diodes, capteurs ou cellules solaires.
